# EUROPÄISCHE PATENTANMELDUNG

(11) **EP 2 927 689 A1**
(43) Veröffentlichungstag der Anmeldung: **07.10.2015**
(21) Anmeldenummer: 15161115.9
(22) Anmeldetag: 26.03.2015
(51) Int. Cl.: G01N 33/543, G01N 21/77, G01N 35/00

(54) **VERFAHREN UND VORRICHTUNG ZUR LABELFREIEN DETEKTION EINES ANALYTEN**

(30) Priorität: 01.04.2014 DE 102014104595
(71) Anmelder: Surflay Nanotec GmbH, 12489 Berlin (DE); Himmelhaus, Michael, 12489 Berlin (DE)
(72) Erfinder: Himmelhaus, Michael, 12489 Berlin (DE); Dähne, Lars, 15366 Hoppegarten (DE); Bischler, Ruben, 14059 Berlin (DE)
(74) Vertreter: Zimmermann & Partner Patentanwälte mbB

(57) **Zusammenfassung**

Es wird ein Verfahren zur labelfreien Detektion eines Analyten und eine zugehörige Analyse-vorrichtung angegeben. Das Verfahren umfasst die Schritte Einströmen eines suspendierten Partikels in einen Mikrokanal eines Mikrochips, Führen des suspendierten Partikels in eine Haltestruktur im Mikrochip und Halten des suspendierten Partikels in der Haltestruktur, optisches Anregen des gehaltenen Partikels und Messen eines ersten Emissionsspektrums des gehaltenen Partikels, Bestimmen einer ersten Resonanzmode des Partikels im ersten Emissionsspektrum, Zuführen des Analyten zu dem gehaltenen Paitikel mittels einer Strömung, erneutes optisches Anregen des gehaltenen Partikels und Messen eines zweiten Emissionsspektrums des gehaltenen Partikels, Bestimmen einer zur ersten Resonanzmode des Partikels korrespondieren zweiten Resonanzmode des Partikels im zweiten Emissionsspektrum, und Bestimmen einer Lageverschiebung, einer Änderung einer Halbwertsbreite und/oder einer Aufspaltung der zweiten Resonanzmode relativ zur ersten Resonanzmode.

## Beschreibung

Die vorliegende Erfindung betrifft ein Verfahren zur labelfreien Detektion eines Analyten, d.h. zur markierungsfreien Detektion des Analyten, an Partikeln, insbesondere Mikropartikeln, über deren optische Resonanzmoden sowie eine zugehörige Vorrichtung.

Markierungsfreie Sensoren zur Detektion bzw. Analyse eines Analyten sind häufig als Oberflächensensoren ausgeführt, bei denen die Oberfläche eines physikalischen Messwandlers derart modifiziert wird, dass die Analyte über einen Messwandler nachweisbar sind. Im Falle bioanalytischer Analysen kann die Oberfläche beispielsweise geeignet biochemisch funktionalisiert werden, um die spezifische Kopplungen mit den gewünschten Liganden zu ermöglichen. Diese Kopplungen können vom Messwandler dann als Änderung in einem mit ihnen verbundenen physikalischen Parameter, wie Masseänderung, Änderung der dielektrischen Eigenschaften, Oberflächenladung, etc., wahrgenommen und in ein elektronisch verarbeitbares Signal gewandelt werden.

Beispiele für derartige Systeme sind die Oberflächenplasmonenresonanz, die Quarzkristall-Mikrowaage, akustische Oberflächenwellen, die Ellipsometrie und die Reflektometrie, wobei die Analyten in der Regel flüssig oder gasförmig sind. Die Anbindung der gesuchten Liganden erfolgt dann typischerweise aus einer Strömung, die über die Oberfläche des Messwandlers geleitet wird. Hierdurch ergibt sich, gerade bei miniaturisierten Systemen, bei denen die Analyten über eine Strömung zum Sensor geführt werden, das Problem, dass die Wechselwirkung zwischen Analyt und Messwandleroberfläche diffusionslimitiert ist, d.h. der Analyt in Obelflächennähe schnell an Liganden verarmt und diese nur durch Diffusion aus dem Fluidvolumen ersetzt werden können. Dies verlängert in der Regel die Messdauer signifikant und wirkt sich letztlich begrenzend auf die Messgenauigkeit aus.

Gleiches gilt für Fälle, in denen kein spezieller Ligand im Analyten gesucht wird, sondern lediglich eine physiko-chemische Eigenschaft des Analyten, wie beispielsweise eine chemische Konzentration oder Komposition, ein Brechungsindex, Viskosität, Dichte, o.ä., bestimmt werden soll. Auch in diesen Fällen kann die Konzentration des Analyten durch Wechselwirkung mit Oberflächen des Systems nahe dem Sensor gegenüber dem Zustand im Inneren des Fluids verändert sein und so das Messergebnis verfälschen.

Optische Resonanzmoden, die im Folgenden auch als Resonanzmoden (kurz: "Moden") bezeichnet werden, können nach optischer Anregung der Mikropartikel durch Interferenz von im Inneren der Mikropartikel vielfach umlaufender optischer Strahlung entstehen. Die Mikropartikel bilden dabei mikroskopische optische Kavitäten, häufig als "Mikroresonatoren" bezeichnet, welche vergleichbar zu den bekannteren Laser-Resonatoren ein charakteristisches optisches Moden-Profil besitzen, das bei geeigneter Anregung beobachtet werden kann. Die sich ausbildenden optischen Resonanzmoden werden dabei von den Eigenschaften der Mikropartikel, wie Material, Form und Oberfläche, festgelegt und von den Umgebungsbedingungen der Mikropartikel beeinflusst, beispielsweise hinsichtlich ihrer Linienbreite, einer etwaigen Aufspaltung und der exakten Lage der Resonanzpositionen. Für den Einsatz als Sensoren sind optische Resonanzmoden- Anregungen in Mikropartikeln von großem Interesse, weil die Moden als Interferenz-Phänomen höchst empfindlich auf Änderungen im Zustand des jeweiligen Mikropartikels und seiner Umgebung reagieren. Dies erlaubt die Entwicklung höchst sensitiver mikroskopischer Messverfahren mit unterschiedlichsten Anwendungen in den Bereichen der physikalischen Messtechnik, der chemischen und biochemischen Analytik. Die Reaktion zwischen Mikropartikeloberfläche und Analyt, die zu einem geeigneten Messwandlersignal umgewandelt werden kann, ist typischerweise schneller und zuverlässiger als mit den oben genannten Nachweisverfahren. Um die Mikropartikel über einen für die Analyse bzw. Detektion hinreichend langen Messzeitraum beobachten zu können, wurden die Mikropartikel bisher bspw. auf einer geeignet vorbehandelten planaren Glasoberfläche immobilisiert, die anschließend zusammen mit den Mikropartikeln geeignet funktionalisiert werden kann. Auf Grund der Immobilisierung wird der für den Analyten zugängliche Teil der Partikeloberfläche jedoch reduziert und somit die Messempfindlichkeit verringert, d.h. insbesondere die Nachweisgrenze für den Analyten zu höheren Konzentrationen verschoben, was durch die Glasvorbehandlung und die nachträgliche Funktionalisierung typischerweise noch verstärkt wird.

Im Hinblick auf das oben Gesagte schlägt die vorliegende Erfindung ein Verfahren gemäß Anspruch 1 und eine Vorrichtung gemäß Anspruch 10 vor.

Gemäß einem Ausführungsbeispiel umfasst ein Verfahren zur labelfreien Detektion eines Analyten die Schritte des Einströmen eines suspendierten Partikels in einen Mikrokanal eines Mikrochips, des Führens des suspendierten Partikels in eine Haltestruktur im Mikrochip und des Haltens des suspendierten Partikels in der Haltestruktur, des optischen Anregens des gehaltenen Partikels und Messens eines ersten Emissionsspektrums des im Mikrochip gehaltenen Partikels, des Bestimmens einer ersten Resonanzmode des Partikels im ersten Emissionsspektrum, des Zuführens des Analyten zu dem im Mikrochip gehaltenen Partikel mittels einer Strömung, des erneuten optischen Anregens des im Mikrochip gehaltenen Partikels und Messens eines zweiten Emissionsspektrums des im Mikrochip gehaltenen Partikels, des Bestimmens einer zur ersten Resonanzmode des Partikels korrespondieren zweiten Resonanzmode des Partikels im zweiten Emissionsspektrum, und des Bestimmen einer Lageverschiebung, einer Änderung einer Halbwertsbreite und/oder einer Aufspaltung der zweiten Resonanzmode relativ zur ersten Resonanzmode.

Dadurch, dass das Partikel, bei dem es sich typischerweise um ein optisch anregbares globuläres Mikropartikel, bzw. sphärisches Mikropartikel, mit einem Durchmesser in einem Bereich von etwa 1 µm bis etwa 50 µm, typischer in einem Bereich von etwa 5 µm bis etwa 15 µm handelt, in einer typischerweise vom Mikrochip vorbestimmten Haltestruktur gehalten wird, jedoch nicht über chemische Bindungen mit einer Kanalwand des Mikrokanals fixiert wird, kann die Messempfindlichkeit des Verfahrens gegenüber einer chemischen Partikelfixierung erhöht werden, da die für den Analyten zugängliche Oberfläche des Partikels beim Halten allenfalls unwesentlich verringert wird. Außerdem kann das Partikel mit einer funktionalisierten Oberfläche (Beschichtung) bereitgestellt werden, ohne dass die Kanalwände des Mikrokanals mitfunktionalisiert werden, wodurch sich die Messempfindlichkeit reduzieren würde. Je nach Analyt bzw. Partikel kann der Mikrokanal und/oder die Zuflüsse dazu auch unabhängig vom Partikel funktionalisiert werden, um eine unspezifische Anlagerung von Analyt bzw. Partikel an den Kanalwänden zumindest zu behindern. Die vorbestimmte Haltestruktur ist typischerweise im Mikrokanal angeordnet oder am Mikrokanal angeordnet und fluidisch mit diesem verbunden, bzw. bildet einen Teil des Mikrokanals. Der Mikrochip kann auch mehrere Mikrokanäle mit jeweiligen Haltestrukturen enthalten.

Der Begriff "Mikrokanal", wie er vorliegend verwendet wird, soll einen Kanal mit einem typischerweise rechteckigen Querschnitt, einer Höhe in einem Bereich von etwa 10 µm bis etwa 250 µm, typischer in einem Bereich von 20 µm bis 100 µm und einer Breite in einem Bereich von etwa 10 µm bis etwa 2500 µm, typischer in einem Bereich von 200 µm bis 1000 µm beschreiben.

Gemäß einer Weiterbildung werden im ersten Emissionsspektrum mehrere erste Resonanzmoden, z.B. zwei erste Resonanzmoden, und im zweiten Emissionsspektrum zu den ersten Resonanzmoden des Partikels korrespondierende zweite Resonanzmoden des Partikels bestimmt, und danach deren Lageverschiebung, Halbwertsbreitenänderung und/oder Modenaufspaltung relativ zur jeweiligen korrespondierenden ersten Resonanzmode bestimmt. Dadurch kann die Messgenauigkeit weiter erhöht werden.

Der Begriff "Analyt", wie er vorliegend verwendet wird, soll eine in einer Flüssigkeit, typischer Weise in einer wässrigen Lösung, lösbare chemische Substanz beschreiben. Beispielsweise kann es sich bei dem Analyten um biologisch und/oder pharmakologisch wirksame Substanzen wie Proteine, Antikörper, Antigene, Hormone, DNS, RNS, Lipide oder auch übliche Medikamente handeln. Es können aber auch rein technisch relevante Analyten sein, wie zum Beispiel anorganische Materialien, Ionen, Schwermetalle und Nanopartikel der verschiedensten Kompositionen oder auch organische Verbindungen, wie zum Beispiel Polymere, Zucker, Rauschgifte. Der Analyt wird mit der strömenden Flüssigkeit dem im Mikrochip gehaltenen Partikel zugeführt und kann dort mit ihm in Wechselwirkung treten. Es kann auch vorgesehen sein, dass mehrere Analyten in der Flüssigkeit gelöst sind, die bspw. an unterschiedlich funktionalisierten und im Mikrochip gehaltenen Partikeln detektiert werden können.

Typischerweise strömt das suspendierte Partikel mit einer Vielzahl anderer suspendierter Partikel, d.h. als Partikelsuspension, in den Mikrokanal ein. Beispielsweise kann die Partikelsuspension mit einer Pumpe in den Mikrokanal gepumpt werden.

Der gelöste Analyt kann mittels einer Pumpe in den Mikrokanal und damit zum gehaltenen Partikel gepumpt werden, wobei bei Verwendung geeigneter Ventile auch die für das Pumpen der Partikelsuspension verwendete Pumpe genutzt werden kann.

Das Führen des suspendierten Partikels kann durch Anströmen des suspendierten Partikels gegen eine im Mikrokanal angeordnete und an die Partikelgröße angepasste trichterförmige oder keilförmige Haltestruktur, die für Partikel, die typischerweise dichter als das suspendierende Medium sind, typischerweise am Boden des Mikrokanals angeordnet ist, erfolgen. Das Partikel kann dann während der Messung durch die Strömung bei typischerweise verringerter Strömungsgeschwindigkeit oder verschwindender Strömungsgeschwindigkeit in der Haltestruktur gehalten und nach der Messung durch Umkehren der Strömungsrichtung wieder aus der Haltestruktur entfernt werden. Vor der Messung des ersten Emissionsspektrums kann zudem das Partikel zum Kanalboden sedimentieren. Dort ist die Strömungsgeschwindigkeit im Mikrokanal geringer, und damit eine höhere Messgenauigkeit zu erwarten.

Alternativ dazu kann das suspendierte Partikel mit der Strömung über eine als Haltestruktur dienende Vertiefung bzw. ein Loch (Sackloch oder Durchgangsloch) im Boden des Mikrokanals geführt werden und anschließend in diese Haltestruktur sedimentieren. In diesen Haltestrukturen kann das Partikel während der Messung zuverlässig und weitgehend unabhängig von der Hauptströmung im Mikrokanal gehalten werden. Dabei kann dem gehaltenen Partikel über die Hauptströmung weiter Analyt zugeführt werden. Typischerweise ist die Vertiefung bzw. das Loch so dimensioniert, dass darin nur ein Partikel gehalten werden kann. Wenn die Haltestruktur als Durchgangsloch (z.B. als Trichter) mit einem minimalen Durchmesser der kleiner als der Durchmesser des Partikels ausgeführt ist, kann das Partikel durch Ansaugen oder Drücken hydrodynamisch in die Haltestruktur geführt werden.

Alternativ oder unterstützend kann das suspendierte Partikel mittels inhomogener elektrischer Felder (typischerweise Wechselfelder im kHz oder MHz-Bereich), die über an Wänden (z.B. am Boden und an der Decke) des Mikrokanals angebrachte Mikroelektroden im Kanal erzeugt werden können, dielektrophoretisch gehalten werden. Mittels der Mikroelektroden des Mikrochips kann die Untersuchungsposition in dieser Haltestruktur auch von den Wänden des Mikrokanals beabstandet sein. Dadurch kann die Messempfindlichkeit weiter erhöht werden. Außerdem kann vorgesehen sein, dass die suspendierten Partikel dielektrophoretisch vereinzelt und/oder in der Strömung des Mikrokanals zur Haltestruktur geführt werden, wozu entsprechende Mikroelektroden an den Wänden des Mikrokanals vorgesehen sein können.

Unterstützend kann ein Ultraschallfeld in den Mikrokanal eingekoppelt werden, um das Vereinzeln und/oder das Führen des Partikels zur bzw. in die Haltestruktur zu erleichtern.

Unterstützend kann die Bewegung eines mechanischen Resonators in den Mikrokanal eingekoppelt werden, um das Vereinzeln und/oder das Führen des Partikels zur bzw. in die Haltestruktur zu erleichtern.

Unterstützend kann ein inhomogenes Magnetfeld im Mikrokanal erzeugt werden, um das Vereinzeln und/oder das Führen des Partikels mit einem magnetisierbaren Kern zur bzw. in die Haltestruktur zu erleichtern.

Unterstützend kann ein fokussiertes elektromagnetisches Feld, typischerweise ein fokussierter Laserstrahl bspw. ein fokussierter Infrarotlaserstrahl, im Mikrokanal erzeugt werden, um das Vereinzeln und/oder das Führen des Partikels zur bzw. in die Haltestruktur zu erleichtern. Als Haltestruktur während der Messung sind sogenannte Laserfallen weniger geeignet, da sie die optischen Eigenschaften des Partikels auf Grund der relativ hohen Leistung verändern können bzw. bei niedrigeren Laserleistungen die Haltekräfte relativ gering sind, was zum Verlust der Partikel führen kann, z.B. beim Schalten einer mit dem Mikrokanal fluidisch gekoppelten Pumpe oder eines Ventils.

Das Partikel weist typischerweise eine hinreichend hohe Transmission von bspw. über 60% in zumindest einem Teil des optischen Spektrums, in dem optische Resonanzmoden angeregt werden sollen, auf. Dieser Teil des optischen Spektrums kann dabei ein Teil des ultravioletten, des sichtbaren, des nahinfraroten oder des infraroten Bereichs des optischen Spektrums sein. Entsprechend können optische Resonanzmoden in verschiedenen Bereichen des optischen Spektrums parallel oder sequenziell im Partikel angeregt werden, wenn das Material in den entsprechenden Teilen der Bereiche des optischen Spektrums die erforderliche geringe Extinktion aufweist.

Das Partikel kann homogen oder heterogen aufgebaut, bspw. mehrschalig aufgebaut sein. So kann ein Kern des Partikels mit einer zusätzlich aufgebrachten optisch aktiven Beschichtung, d.h. mit einer die Anregung der optischen Resonanzmoden beeinflussenden Beschichtung, und/oder einer funktionalen Beschichtung, d.h. einer die Wechselwirkung des Partikels mit dem Analyten beeinflussenden Beschichtung, versehen sein. Beispielsweise kann eine (spiegelnde) Metallbeschichtung als optisch aktive Beschichtung des Partikels die Entstehung von radial verlaufenden optischen Resonanzmoden, sog. "Fabry-Perot-Moden", beeinflussen. Die Aufbringung einer funktionalen Beschichtung von spezifisch wechselwirkenden Proteinen hingegen hat in der Regel keinen Einfluss auf Existenz und Art der optischen Resonanzmoden, sondern ist lediglich für den Einsatz der Partikel als spezifische Mikrosensoren im Bereich der Bioanalytik von Bedeutung. Die funktionale Beschichtung kann außerdem so ausgeführt sein, dass sie mit verschiedenen Analyten wechselwirken kann, z.B. mit verschiedenen Antigenen. Dies ermöglicht den Nachweis bzw. sogar die Quantifizierung unterschiedlicher Analyten mittels eines gehaltenen Partikels.

Die Form des Partikels kann regulär, aber auch irregulär sein. Bei irregulärer Form sind die Moden bezüglich Existenz, Position, Linienbreite und Polarisation jedoch nicht so definiert vorhersagbar und können von Partikel zu Partikel variieren. Unter den regulären Formen, wie z. B. Polyeder oder Ellipsoiden, sind daher insbesondere Partikel von hoher Symmetrie von Interesse, wie beispielsweise sphärische Mikropartikel. Symmetriebedingt sind hier in unterschiedlichen Raumrichtungen propagierende optische Resonanzmoden entartet, d.h. sie weisen (bei homogener Umgebung) identische Modenpositionen und Linienbreiten auf, wodurch sich die beobachtbaren Spektren deutlich vereinfachen. Dies ist für Anwendungen im Bereich der optischen Sensorik häufig vorteilhaft. Insbesondere lassen sich so auch leichter Rückschlüsse auf inhomogen wirkende Einflüsse auf die Partikel oder ihre Umgebung ziehen, wenn eine Aufhebung der Entartung beobachtet wird.

Für die Verwendung der Partikel als optische Mikrosensoren kann sowohl eine mikroskopisch raue als auch eine glatte äußere Oberfläche vorteilhaft sein, je nach Art der Oberfläche und der sich ausbreitenden Moden. Für optisch aktive Metallbeschichtungen kann eine raue Oberfläche die Anregung von Oberflächenplasmonen begünstigen, die mit ihren starken Nahfeldeffekten zum Sensorsignal beitragen. Für unbeschichtete dielektrische Partikel oder dielektrische Partikel mit einer funktionalen Beschichtung ist es häufig wünschenswert, die Oberflächenstreuung so gering wie möglich zu halten, was durch eine möglichst glatte Oberfläche erreicht werden kann.

Je nach Beschaffenheit der Mikropartikel bzgl. Material, Form und Oberfläche sind die anregbaren optischen Resonanzmoden unterschiedlich. Ein hilfreiches Kriterium zu ihrer Klassifizierung ist dabei die Art und Weise, in der vom Inneren her Photonen an der Partikeloberfläche reflektiert werden. Wie oben bereits erwähnt, ist es wünschenswert, wenn das Innere der Partikel im gewählten Arbeitsbereich des elektromagnetischen Spektrums eine hinreichend hohe Transmission aufweist, um eine freie Propagation der elektromagnetischen Strahlung im Inneren zu gewährleisten. An der Innenseite der Partikeloberfläche sollte die Strahlung möglichst effizient reflektiert werden, um im Inneren des Mikropartikels zu verbleiben und durch Vielfachreflexion und Selbstinterferenz schließlich zur Ausbildung von Resonanzmoden zu führen. Nach einem bekannten physikalischen Prinzip (z. B. Fourieroptik) ist die Linienbreite der Moden dabei um so schmaler, je länger die Strahlung im Partikel verbleibt, d.h. je länger der Weg ist, den die Strahlung dabei im Partikel zurücklegt.

Die Reflexion an der Oberfläche des Partikels kann - je nach ihrer Beschaffenheit - unterschiedlich erfolgen. Bei metallischen Oberflächen, beispielsweise, erfolgt die Reflexion am effizientesten in normaler Richtung zur Oberfläche. Dies begünstigt die oben bereits erwähnte Ausbildung von radialen Fabry-Perot Moden. Bei dielektrischen, homogenen Mikropartikeln hingegen erfolgt die Reflexion am effizientesten unter streifendem Einfall zur Oberfläche. Dies begünstigt die Ausbildung von optischen Resonanzmoden, die an der Oberfläche entlang wandern. Besonders effizient ist diese Art der Modenpropagation, wenn der Brechungsindex des Partikels größer als der seiner Umgebung ist. Dann herrscht ab einem bestimmten Grenzwinkel Totalreflexion im Inneren des Partikels und es bilden sich um den Partikeläquator umlaufende Moden hoher Güte, d.h. kleiner Linienbreite, aus. Diese Moden sind insbesondere in dielektrischen sphärischen Mikropartikeln gut zu beobachten und werden in der Literatur häufig als Whispering Gallery Modes (WGMs) bezeichnet.

Die Formulierung "Resonanzmode des Partikels", wie sie vorliegend verwendet wird, soll eine optische Resonanzmode, insbesondere eine Whispering Gallery Mode des Partikels, aber auch andere optische Resonanzmoden, wie beispielsweise Fabry-Perot-Moden oder irreguläre Moden in irregulär geformten Partikeln, beschreiben.

Die Resonanzmoden können auf verschiedene Weisen im Partikel erzeugt werden. Eine sehr elegante Möglichkeit ist die Anregung aus dem Partikelinneren heraus, bei der bspw. ein intrinsischer oder extrinsischer Fluorophor genutzt bzw. in das Partikel eingebracht wird. Unter einem intrinsischen Fluorophor ist hierbei ein strahlender Anregungszustand des Materials zu verstehen, aus dem das Partikel besteht (im Innern und/oder an der Oberfläche), welcher dann zur Emission von Strahlung im gewünschten Arbeitsbereich der Resonanzmodenanregung führt. Dies kann über Lumineszenz, Phosphoreszenz, typischerweise Fluoreszenz, oder Raman-Anregung geschehen. Ein extrinsischer Fluorophor, wie ein organischer Farbstoff oder Halbleiter-Quantenpunkt, kann dagegen während oder nach der Herstellung der Mikropartikel in das Material eingebracht oder seine Oberfläche angebracht werden.

Unter dem Begriff "Emissionsspektrum", wie er vorliegend verwendet wird, wird ein Lumineszenzspektrum, typischerweise ein Fluoreszenzspektrum, verstanden.

Die Anregung des Partikels, typischerweise seiner Fluorophore, erfolgt optisch von außen. Das Material des Mikropartikels sollte daher für die Anregungswellenlänge ausreichend transparent sein (> 10% Transmission beim Durchgang durch das Partikel), um zu hohe Absorption und damit verbundene thermische Effekte zu vermeiden. Die angeregten Fluorophore emittieren dann bei einer anderen Wellenlänge, die je nach Funktionsprinzip entweder bei höheren (down conversion) oder niedrigeren (up conversion) Wellenlängen liegen kann. Aus dieser in der Regel recht breiten Emissionsbanden (typischerweise mehr als 100 nm für organische Farbstoffe) bilden sich dann die dem jeweiligen Mikropartikeltyp und den Umgebungsbedingungen entsprechenden schmalbandigeren Resonanzmoden heraus (typischerweise 50 bis 1000 pm Halbwertsbreite).

Alternativ dazu kann eine Anregung auch ohne Fluorophor über andere bekannte Anregungsmechanismen erfolgen, wie beispielsweise über Nahfeldkopplung mittels eines optischen Kopplers (z.B. eines Prismas oder eines erodierten Lichtwellenleiters) oder eines stark fokussierten Laserstrahls. In diesen Fällen wird typischerweise mit einer durchstimmbaren schmalbandigen Anregungsquelle gearbeitet, um nur eine einzelne oder wenige Resonanzmoden anzuregen und zu verfolgen.

Eine weitere Alternative stellt die Anregung von optischen Resonanzmoden über Mie-Streuung an den Mikropartikeln dar. Dieser Prozess ist jedoch weniger effizient.

Das erste und zweite Emissionsspektrum werden typischerweise mit demselben optischen Analysator, typischerweise einem Spektrometer, gemessen. Alternativ dazu kann ein Interferometer, bspw. ein auf die erwartete Resonanzmode abgestimmtes Etalon zur Messung der Emissionsspektren verwendet werden. Typischerweise werden das erste und zweite Emissionsspektrum mit einem spektralen Auflösungsvermögen (λ/Δλ) von zumindest 10⁴ gemessen. Dies entspricht bei einer Wellenlänge λ von 500 nm einer optischen Auflösung Δλ von zumindest 50 pm (Picometer), z.B. 25 pm oder sogar besser, z.B. 12 pm. Dies ermöglicht eine äußerst genaue Bestimmung der Resonanzmoden im ersten und zweiten Emissionsspektrum.

Typischerweise werden zur optischen Anregung und/oder zur Auskoppelung der vom im Mikrochip gehaltenen Partikel emittierten optischen Strahlung entsprechende Hilfsoptiken verwendet.

Typischerweise sind die Hilfsoptiken zudem so ausgeführt, dass immer nur ein gehaltenes Partikel angeregt und vermessen werden kann.

Auf Grund ihrer Schmalbandigkeit lassen sich die Lagen und Bandbreiten der Resonanzmoden im ersten und zweiten Emissionsspektrum sehr genau bestimmen.

Da das erste Emissionsspektrum vor dem Zuführen des Analyten gemessen wird, kann es als Referenzspektrum für das zweite Emissionsspektrum desselben Partikels, das nach dem Zuführen des Analyten gemessen wird, genutzt werden. Aus einem sich anschließenden Vergleich einer oder mehrerer Resonanzmoden des zweiten Emissionsspektrums (zweite Resonanzmoden) mit einer jeweils entsprechenden Resonanzmode des ersten Emissionsspektrums (erste Resonanzmoden) kann der Analyt hochempfindlich nachgewiesen werden. Dies kann typischerweise sogar in Echtzeit erfolgen.

Der Vergleich der ersten und zweiten Resonanzmoden kann die Bestimmung einer Lageverschiebung (Frequenzverschiebung), der Änderung der Halbwertsbreiten und/oder die Bestimmung einer Aufspaltung der Resonanzmoden, wie sie bspw. bei einer Formänderung des Partikels auftreten kann, umfassen.

Aus dem Vergleich der ersten und zweiten Resonanzmoden kann die Menge des auf dem im Mikrochip gehaltenen Partikel adsorbierten Analyten berechnet werden und damit auf die Konzentration des Analyten im Fluid geschlossen werden.

Gemäß einer Weiterbildung werden nach dem Messen des zweiten Emissionsspektrums ein oder sogar mehrere weitere Emissionsspektren des im Mikrochip gehaltenen Partikel gemessen, wobei jeweils ein erneutes optisches Anregen des im Mikrochip gehaltenen Partikels und Messen des weiteren Emissionsspektrums des im Mikrochip gehaltenen Partikels erfolgt. Danach kann eine zur ersten Resonanzmode des Partikels bzw. zur zweiten Resonanzmode des Partikels korrespondierende jeweilige weitere Resonanzmode des Partikels im weiteren Emissionsspektrum, sowie eine Lageverschiebung, eine Änderung einer Halbwertsbreite und/oder eine Aufspaltung der weiteren Resonanzmode relativ zur ersten Resonanzmode bzw. zweiten Resonanzmode erfolgen. Daraus kann eine Adsorptionskinetik des Analyten an dem im Mikrochip gehaltenen Partikel bestimmt werden. Wie oben für die erste Resonanzmode und zweite Resonanzmode erläutert, können zur weiteren Erhöhung der Messgenauigkeit auch mehrere jeweilige weitere Resonanzmoden im weiteren Emissionsspektrum bestimmt und mit korrespondierenden ersten bzw. zweiten Resonanzmoden verglichen werden.

Vor dem Messen des ersten Emissionsspektrums des im Mikrochip gehaltenen Partikels kann eine Detektion des im Mikrochip gehaltenen Partikels bzw. der im Mikrochip gehaltenen Partikel erfolgen, bspw. über ein 2D-Bild des Mikrokanals mit der Haltestruktur bzw. den Haltestrukturen, welches mittels einer Mikroskopoptik und einer Digitalkamera aufgenommen werden kann. Eine damit verbundene Überprüfung, ob die Partikel erfolgreich gehalten werden, kann besonders dann hilfreich sein, wenn eine Vielzahl von Partikeln in jeweiligen Haltestrukturen gehalten werden sollen. Dies vermeidet Fehlmessungen in nichtbeladenen Halte strukturen.

Außerdem kann vor dem Messen des ersten Emissionsspektrums des im Mikrochip gehaltenen Partikels vorgesehen sein, die Untersuchungsposition des gehaltenen Partikels in seiner Haltestruktur genauer zu bestimmen. Dies ist bspw. für in zylindrische Löcher sedimentierte und dort gehaltene Partikel wichtig, deren genaue Position im Loch variieren kann, wenn es einen größeren Durchmesser als das darin gehaltene Partikel aufweist. Die Bestimmung der Position des Partikels in der Haltestruktur kann über eine Maximierung der Signalintensität des Emissionsspektrums des gehaltenen Partikels und/oder die Auswertung eines 2D-Bildes des Mikrokanals mit der Haltestruktur erfolgen.

Gemäß noch einer Weiterbildung wird das Verfahren nach der Detektion bzw. Analyse des Analyten und einem optionalen Spülschritt des Mikrokanals und des gehaltenen Partikels mit dem Zuführen eines weiteren Analyten und der Bestimmung entsprechender Resonanzmoden fortgesetzt.

Da das bzw. die Partikel im Mikrochip nur temporär gehalten werden, können sie nach der Messung wieder entfernt, typischerweise ausgespült werden. Dieser Prozess kann durch die Einkoppelung von mechanischen Schwingungen, eines Ultraschallfeldes und/oder von Magnetfeldern, elektrischen Feldern und/oder elektromagnetischen Feldern (Laserpinzetten) unterstützt werden. Dies ermöglicht eine Wiederverwendung des Mikrochips für nachfolgende Untersuchungen.

Gemäß einem Ausführungsbeispiel umfasst eine Vorrichtung zur labelfreien Detektion eines Analyten eine Fluidikeinheit umfassend eine Dosiereinheit, einen Mikrochip umfassend einen Mikrokanal, der mit der Dosiereinheit derart verbunden ist, dass eine Lösung mit dem Analyten und eine Suspension von Partikeln in den Mikrokanal gefördert werden kann, und eine im oder am Mikrokanal angeordnete Haltestruktur für die Partikel, eine Lichtquelle zur Anregung einer Resonanzmode eines in der Haltestruktur gehaltenen Partikels, einen Spektralanalysator zur Messung eines Emissionsspektrums des in der Haltestruktur gehaltenen Partikels; und eine Steuer- und Auswerteeinheit, die mit der Lichtquelle, dem Spektralanalysator und der Fluidikeinheit verbunden ist und eingerichtet ist, eine Resonanzmode im Emissionsspektrum zu bestimmen.

Die Dosiereinheit kann eine oder mehrere Pumpen, eine oder mehrere Ventile, und einen oder mehrere Schläuche umfassen, um den Mikrokanal fluidisch mit der einen oder mehreren Pumpen über einen oder mehrere Anschlüsse des Mikrochips zu verbinden.

Die Verwendung des Mikrochips mit einer oder mehreren, typischerweise in einem Raster angeordneten Haltestrukturen für die Partikel, in denen die Partikel nicht permanent fixiert sind, sondern während der Messung räumlich gut lokalisiert, aber beweglich sind und daher weitgehend ungestört mit dem Analyten oder mehreren Analyten der Lösung wechselwirken können, ermöglicht eine hochempfindliche und robuste Detektion bzw. Analyse der Wechselwirkung und damit des bzw. der Analyten.

Dazu ist die Steuer- und Auswerteeinheit typischerweise eingerichtet, eines der oben erläuterten Verfahren auszuführen.

So kann die Steuer- und Auswerteeinheit eingerichtet sein, folgende Schritte auszuführen: Einströmen der Suspension in den Mikrokanal, Führen des suspendierten Partikels in die Haltestruktur und Halten des suspendierten Partikels in der Haltestruktur, optisches Anregen des in der Haltestruktur gehaltenen Partikels und Messen eines ersten Emissionsspektrums des in der Haltestruktur gehaltenen Partikels, Bestimmen einer ersten Resonanzmode des Partikels im ersten Emissionsspektrum, Führen des Analyten zu dem in der Haltestruktur gehaltenen Partikel mittels einer Strömung der Lösung, erneutes optisches Anregen des in der Haltestruktur gehaltenen Partikels und Messen eines zweiten Emissionsspektrums des in der Haltestruktur gehaltenen Partikels, Bestimmen einer zur ersten Resonanzmode des Partikels korrespondieren zweiten Resonanzmode des Partikels im zweiten Emissionsspektrum, und Bestimmen einer Lageverschiebung, einer Aufspaltung und/oder einer Änderung einer Halbwertsbreite der zweiten Resonanzmode relativ zur ersten Resonanzmode. Aus einem anschließenden Vergleich der Resonanzmoden kann die Steuer- und Auswerteeinheit dann typischerweise eine Analyse der Wechselwirkung von Analyt und Partikel vornehmen.

Der Spektralanalysator ist typischerweise ein hochauflösendes Spektrometer mit einem Gittermonochromator mit einer Liniendichte von mindestens 1200/mm oder sogar mindestens 2400/mm. Das Spektrometer kann einen Bildsensor mit mindestens 1000 Pixeln (je Dimension) und mit einer Pixelbreite von höchstens 24 µm oder sogar höchstens 10 µm, z.B. eine entsprechende Zeilen-CCD oder eine gebinnte 2D-CCD oder eine Photodiode und einen zwischen dem Gittermonochromator und der Photodiode angeordneten Spalt von maximal 100 µm aufweisen. Bei dem Spektralanalysator kann es sich aber auch um ein an die erwarteten Resonanzmoden angepasstes Interferometer, z.B. ein entsprechendes Etalon, handeln.

Zwischen der Lichtquelle, die typischerweise ein Laser oder eine lichtemittierende Diode (LED) ist, und dem Mikrochip, dessen Mikrokanal typischerweise zumindest im Bereich der Haltestruktur bzw. der Haltestrukturen von einem optisch transparenten Boden und/oder einer optisch transparenten Decke begrenzt wird, und/oder zwischen dem Spektralanalysator und dem Mikrochip ist typischerweise eine Optik angeordnet. Die Optik dient zum Einkoppeln des Lichts der Lichtquelle in den Mikrochip (zum Anregen des in der Haltestruktur gehaltenen Partikels) und zum Sammeln und Weiterleiten von Licht, das von dem in der Haltestruktur gehaltenen und optisch angeregten Partikel emittiert wird, zum Spektralanalysator.

Der Begriff "Mikrochip", wie er vorliegend verwendet wird, soll einen Chip mit einem optisch zumindest teilweise zugänglichen Mikrokanal, der über einen Einlass mit einer Flüssigkeit bzw. einer Suspension beladen werden kann, beschreiben. Die Begriffe Mikrochip und Mikrofluidik-Chip werden vorliegend synonym benutzt.

Bei der Haltestruktur kann es sich um ein Loch im Boden des Mikrokanals mit einem Durchmesser von typischerweise weniger als 30 µm handeln.

Alternativ dazu kann die Haltestruktur auch von zwei am Boden des Mikrokanals angeordneten und voneinander beabstandeten Barrieren gebildet werden, die einen minimalen Abstand zueinander aufweisen, der kleiner als das zu haltende Partikel ist. Der minimale Abstand der Barrieren ist typischerweise kleiner als 20 µm, noch typischer in einem Bereich von etwa 1 µm bis 9 µm. In einer Aufsicht können die beiden Barrieren eine trichterförmige Haltestruktur bilden.

Die Begriffe "trichterförmige Haltestruktur" und "trichterförmige Struktur", wie sie vorliegend verwendet wird, sollen eine Struktur beschreiben, die in einer Querschnittsansicht durch die Struktur zwei Teile aufweist, deren Abstand zueinander entlang einer Symmetrieachse zwischen ihnen zwischen einem minimalen Wert und einem maximalen Wert variiert. In einem Mikrochip kann der minimale Wert der trichterförmigen Haltestruktur kleiner als der Durchmesser der zur Messung vorgesehenen Partikel und der maximale Wert der trichterförmigen Haltestruktur größer als der Durchmesser gewählt werden. Er wird typischerweise jedoch nicht größer als der doppelten Durchmesser des Partikels gewählt, damit jeweils nur ein Partikel in der Haltestruktur festgehalten werden kann. Beispielsweise kann der minimale Wert in einem Bereich von etwa 1 bis 15 µm liegen und der maximale Wert zwischen 2 bis 30 µm liegen.

Weitere vorteilhafte Ausgestaltungen, Einzelheiten, Aspekte und Merkmale der vorliegenden Erfindung ergeben sich aus den Unteransprüchen, der Beschreibung sowie den beigefügten Zeichnungen. Darin zeigt:
Fig. 1 eine schematische Darstellung einer Vorrichtung zur labelfreien Detektion eines Analyten mit einem Mikrochip gemäß einem Ausführungsbeispiel;
Fig. 2 eine Darstellung eines Mikrochips mit einem Mikrokanal, der in der in Fig. 1 dargestellten Vorrichtung als Mikrochip verwendet werden kann, gemäß einem Ausführungsbeispiel;
Fig. 3A eine Querschnittsdarstellung durch einen Mikrochip mit einem Mikrokanal und mehreren im Boden des Mikrokanals angeordneten trichterförmigen Haltestrukturen gemäß einem Ausführungsbeispiel;
Fig. 3B am Boden eines Mikrokanals angeordnete Haltestrukturen in einer Aufsichtsansicht gemäß Ausführungsbeispielen;
Fig. 4 gemessene Emissionsspektren eines in einer Haltestruktur gehaltenen Partikels zur Bestimmung von spezifischen Streptavidin-Biotin-Wechselwirkungen, die mit einer Vorrichtung zur labelfreien Detektion eines Analyten erhalten wurden, wie sie in Figur 1 dargestellt ist; und
Fig. 5 die Lageverschiebung der Resonanzmoden in den in Fig. 4 gezeigten Emissionsspektren.

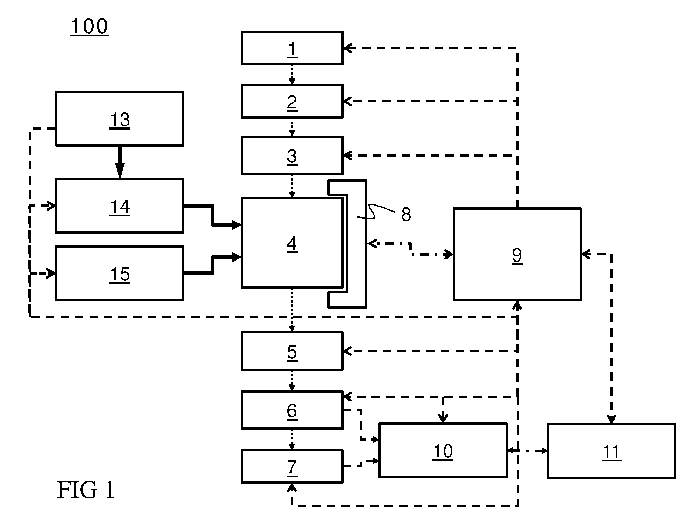

Figur 1 zeigt in einer schematischen Darstellung den Aufbau einer exemplarischen Vorrichtung (Messsystem) 100 zur labelfreien Detektion von Analyten unter Verwendung optischer Sensoren auf Basis von für optische Resonanzmoden-Anregungen geeigneten Mikropartikeln, im Folgenden kurz auch als "Mikropartikelsensoren" bezeichnet. In Figur 1 werden mögliche Wechselwirkungen der Komponenten der Vorrichtung durch Pfeile symbolisiert, wobei Flüsse von Steuerungsinformationen und Messdaten durch gestrichelte Pfeile und der Weg des Lichts durch gepunktete Pfeile angezeigt werden. Die Vorrichtung 100 weist einen Mikrochip (Mikrofluidik-Chip) 4 mit einem nicht dargestellten Mikrokanal auf. Wie weiter unten im Detail ausgeführt, erlaubt der Mikrofluidik-Chip 4 den Betrieb des mindestens einen Mikropartikelsensors ohne dessen permanente Fixierung, so dass letzterer bei Bedarf jederzeit einfach ausgetauscht werden kann. Dies ermöglicht den fortwährenden Betrieb der Vorrichtung 100 auch nach Erschöpfung eines einzelnen Mikropartikelsensors und ermöglicht so den Einsatz der Vorrichtung u. a. auch in den Bereichen, die eine permanente Überwachung der zu messenden Größen erfordern. Dies kann beispielsweise bei der Wasser-/Abwasser- Kontrolle, der Überwachung auf biochemische, chemische, bakterielle oder virale Kontamination, der Prozess-Kontrolle in Produktionsprozessen und dergleichen der Fall sein.

Der Mikrofluidik-Chip 4 ist zu seinem Betrieb an eine steuerbare Dosiereinheit 14 angeschlossen, die die Versorgung mit den für die jeweilige Messaufgabe erforderlichen Medien sicherstellt. Hierzu gehören neben dem Fluid, in dem der Mikropartikelsensor suspendiert und betrieben werden soll, auch die Mikropartikelsensoren selbst. Typischerweise umfasst die Dosiereinheit 14 eine oder mehrere Pumpen, Schläuche und Ventile. Optional kann eine durch den Nutzer des Systems separat bedienbare manuelle Dosiereinheit 13 zur manuellen Zuführung der Medien, des Analyten oder der Mikropartikelsensoren zum Mikrochip oder zur steuerbaren Dosiereinheit 14 vorgesehen sein.

Die Dosiereinheit 14 kann so ausgelegt werden, dass sie die Positionierung der Mikropartikelsensoren, d.h. das Führen der Mikropartikelsensoren in jeweilige Haltepositionen, und den Austausch der Mikropartikelsensoren im Mikrofluidik-Chip 4 übernehmen kann. Optional kann vorgesehen sein, unterstützend eine zusätzliche aktive Mikropartikelkontrolleinheit 15 einzusetzen, bei der zusätzlich Kräfte auf die Mikropartikelsensoren vor, während oder nach der Messung ausgeübt werden können. Bspw. kann die aktive Mikropartikelkontrolleinheit 15 einen Hochfrequenzgenerator zur Einkoppelung elektrischer Felder in den Mikrokanal umfassen, um dielektrophoretische Kräfte auf die Mikropartikelsensoren ausüben zu können.

Die bisher vorgestellten Komponenten gewährleisten die Bereitstellung der Mikropartikelsensoren selbst sowie die Konditionierung der gewünschten Messaufgabe. Die übrigen Komponenten der Vorrichtung 100 dienen im Wesentlichen der Anregung und Auslese der optischen Resonanzmoden, ihrer Analyse und Interpretation in Bezug auf die definierte Messaufgabe. Eine Ausnahme macht dabei lediglich die digitale Steuerung 9, die die gesamte Vorrichtung 100 inkl. der bereits beschriebenen Komponenten steuert und darüber hinaus zusammen mit einer Eingabe-Ausgabeeinheit 11 die Kommunikation mit dem Nutzer gewährleistet.

Die Eingabe-Ausgabeeinheit 11 dient der Kommunikation mit dem Nutzer und teilt insbesondere das (laufende) Ergebnis der Messung mit. Neben einer visuellen oder akustischen Ausgabe zur Ablese durch den Nutzer kann die Übergabe der Information auch über eine geeignete Schnittstelle an eine übergeordnete Steuerung erfolgen, wie dies beispielsweise für den Einsatz der Vorrichtung als in-line Mess-System in der Prozessüberwachung wünschenswert ist.

Die digitale Steuerung 9 koordiniert typischerweise den gesamten Messablauf, insbesondere die Abläufe zwischen Mikrofluidik (z. B. Analyt-Dosierung, Spülen, Injektion von Mikropartikel-Sensoren, etc.) und Optik (z. B. Anregung und Detektion von optischen Resonanzmoden), digitaler Signalverarbeitung und der Eingabe-Ausgabeeinheit. Sie sendet Befehle an und empfängt Rückmeldungen von den diversen Teilsystemen, die sie kontrolliert, überwacht und steuert.

Die optischen Resonanzmoden werden in den Mikropartikelsensoren mit Hilfe der Komponenten 1, 2, 3 und 8 angeregt. Über die Komponenten 5, 6 und 7 werden diese dann optisch detektiert und in elektronisch verarbeitbare Information umgewandelt, die von der digitalen Signalverarbeitung 10 aufbereitet und analysiert und schließlich als für den Nutzer verwertbare Information im Sinne seiner Fragestellung von der Eingabe-Ausgabeeinheit 11 ausgegeben wird. Die digitale Steuerung 9 und die digitale Signalverarbeitung 10 bilden zusammen eine Steuer- und Auswerteeinheit 9, 10. Die Funktionen der digitalen Steuerung 9, der digitalen Signalverarbeitung 10 und der Eingabe-Ausgabeeinheit 11 können von einem entsprechend ausgerüsteten PC oder einer Workstation, aber auch von einem Tablet-Computer oder einem Mobiltelefon (Smartphone) bereitgestellt werden.

Die jeweiligen Komponenten werden im Folgenden kurz beschrieben. Als anregende Lichtquelle 1 kann jede Lichtquelle dienen, die zumindest in einem Teil des optischen Anregungsbereichs der optischen Resonanzmoden der Mikropartikel emittiert. Die Wahl ist damit von der Wahl der eingesetzten Mikropartikel und der Art ihrer Anregung abhängig. Es können thermische Strahler (Kaltlichtquellen, Halogenlampen, UV-Lampen, Quecksilberdampflampen, etc.), LEDs und Laser eingesetzt werden. In der Regel lassen sich dabei inkohärente Quellen, wie thermische Strahler und LEDs, weniger gut fokussieren als kohärente Quellen, wie Laser. Dadurch wird auf dem Mikrochip in der Regel ein größerer Bereich gleichzeitig bestrahlt, so dass typischerweise mehrere Mikropartikel gleichzeitig angeregt werden. Dadurch können mehrere Mikropartikel gleichzeitig als Sensoren dienen, was jedoch nur für eine begrenzte Anzahl von Anwendungen Vorteile verspricht. Bei hochempfindlichen Messungen sind Laser im Vorteil, die punktgenau auf ein einzelnes Mikropartikel fokussiert werden können und dann nur dieses anregen. Entsprechend benötigen inkohärente Quellen in der Regel eine höhere optische Ausgangsleistung, um die gleiche Leistungsdichte auf dem Mikropartikel zu erzeugen, sind dafür aber in der Anschaffung günstiger.

Ein im Strahlengang angeordneter Shutter (Verschluss) 2 dient der schnellen Unterbrechung der optischen Anregung durch Blockieren des Lichtwegs von der Lichtquelle 1 zum Mikrochip 4. Auf diese Weise kann die Expositionszeit der Mikropartikelsensoren reduziert werden. Dies vermeidet zu lange Belichtungszeiten der eingesetzten Mikropartikel, die zu Veränderungen der Mikropartikel führen können, die sich wiederum auf die optischen Resonanzmoden und damit schließlich auf das Messergebnis auswirken können.

Da es sich bei den Mikropartikelsensoren um mikroskopisch kleine Partikel handelt, ist ihre Anregung typischerweise nur mit Hilfe einer entsprechenden, strahlreduzierenden Koppel-Optik 3 effizient. Dies ist insbesondere dann wichtig, wenn der Mikrochip 4 mehr als nur eine Haltevorrichtung enthält und bei direkter Einstrahlung des von der Lichtquelle 1 kommenden anregenden Lichts die Gefahr besteht, dass mehr als nur ein gehaltener Mikropartikelsensor angeregt wird. Eine typische Koppel-Optik 3 besteht aus Elementen zur Strahlaufbereitung der Lichtquelle 1, wie z. B. dielektrischen und/oder räumlichen Filtern, Kollektoren, Kollimatoren und/oder Teleskopen zur Anpassung von Strahlquerschnitt und Strahldivergenz. Die Einkopplung des Lichts in das Partikel kann über ein Mikroskop-Objektiv bewerkstelligt werden. Je nach Auslegung der Optik kann der Lichtstrahl vom Mikroskop-Objektiv auf das Mikropartikel fokussiert oder kollimiert auftreffen.

Auf Grund der geringen Größe der Mikropartikel ist ihre Positionierung im optischen Strahlengang sowohl anregungs- als auch detektionsseitig wünschenswert, um eine optimale Signalstärke der optischen Resonanzmoden zu gewährleisten. Die alleinige Positionierung mit der Haltevorrichtung bzw. der Haltestruktur des Mikrofluidik-Chips kann dabei zu ungenau sein. Daher ist der Mikrofluidik-Chip 4 typischerweise auf einer fein positionierbaren xyz-Verstelleinheit (xyz-Tisch) 8 montiert, die im Regelkreis mit der digitalen Signalverarbeitung 10 und der digitalen Steuerung 9 des Messablaufs zusammen das jeweils ausgewählte Mikropartikel in allen drei Achsen, also lateral zur und entlang der optischen Achse des optischen Systems, auf optimale Resonanzmoden-Anregung und -signalstärke hin feinjustieren kann. Alternativ dazu kann auch der Mikrofluidik-Chip 4 fixiert und das optische System relativ dazu positioniert werden. Dies ist typischerweise wegen der dadurch leicht entstehenden Bildfehler im optischen Strahlengang jedoch aufwendiger. Außerdem können so nur schlecht weitere Haltevorrichtungen auf dem Mikrofluidik-Chip angefahren werden. Eine andere Möglichkeit ist, die laterale Positionierung durch Verschieben des Mikrofluidik-Chips zu realisieren, während die Justage in der optischen Achse von einer oder beiden Koppel-Optiken 3 und/oder 5 übernommen wird.

Wie schon für die Anregung, wird aufgrund der geringen Partikelgröße typischerweise auch detektionsseitig eine entsprechende hochauflösende Koppel-Optik 5 eingesetzt, um eine zuverlässige Detektion der angeregten optischen Resonanzmoden zu gewährleisten. Weil die Mikropartikel in der Regel nahezu isotrop in alle Raumrichtungen abstrahlen, ist hier eine Koppel-Optik 5 mit hoher numerischer Apertur (N.A.) besonders vorteilhaft. Typischerweise liegt die N.A. bei > 0.5. Wie in der Raman- oder Fluoreszenzmikroskopie üblich, können die Koppel-Optiken 3 und 5 teilweise dieselben optischen Komponenten, wie beispielsweise ein gemeinsames Mikroskop-Objektiv mit hoher N.A., benutzen. Dies ist besonders kosteneffizient und raumsparend, bedarf aber zusätzlicher optischer Elemente zur Strahlteilung und-filterung, da das Anregungslicht von dem von den Mikropartikeln emittierten Licht separiert werden muss. Hier können geeignete, von den genannten Spektroskopien benutzte Verfahren, wie beispielsweise (dielektrische oder Bragg-) Strahlteiler und Filter (auch sog. Notch-Filter), eingesetzt werden.

Zur Bestimmung der optischen Resonanzmoden nach Lage (z. B. Vakuum-Wellenlänge, Frequenz oder Wellenzahl), Breite (z. B. Halbwertsbreite, FWHM von engl. Full Width at Half Maximum) und/oder Aufspaltung (z. B. bei inhomogener Umgebung oder Deformation des Mikropartikels aufgrund von äußeren Kräften) wird eine Spektralanalysatoreinheit 6, 7 mit einem Spektral-Analysator 6 verwendet. Dazu wird das vom Mikropartikelsensor emittierte und von der Koppel-Optik 5 aufgefangene Licht mit Hilfe diffraktiver und/oder interferometrischer Methoden analysiert.

Typischerweise wird dabei in Abhängigkeit von der Wellenlänge des einfallenden Lichts ein Intensitätsmuster erzeugt oder eine bestimmte Raumrichtung ausgeleuchtet, so dass mit einem Detektor 7 der Spektralanalysatoreinheit 6, 7 ein messbarer Zusammenhang zwischen Wellenlänge und ihrer jeweiligen Intensität entsteht. Dies kann beispielsweise mit Hilfe eines Spektrometers, Spektrographen, Interferometers 6 oder eines Interferenzfilters 6 als Spektral-Analysator 6 geschehen. Wichtig ist dabei, dass im gewünschten Arbeitsbereich, also dem für die Sensorinformation benutzten Teil des Emissionsspektrums der Mikropartikel, über einen ausreichend weiten Spektralbereich hinweg das Emissionsspektrum mit ausreichend hoher Auflösung bestimmt werden kann. Insbesondere bei interferometrischen Methoden kann dies eine algorithmische Nachbearbeitung der vom Detektor 7 gemessenen Signale (z. B. Intensitätsmuster) erfordern. Bei diffraktiven Systemen, wie einem Spektrometer oder Spektrographen, werden diese Größen (Detektionsbereich und Auflösung) über Parameter wie Spaltgrößen, fokale Längen, Pixelgrößen, Größe von Bildsensoren und/oder Liniendichten der diffraktiven Elemente (z. B. Reflexionsgitter) festgelegt.

Ein Beispiel für eine ausreichend hohe optische Auflösung ist ein Wert von besser als 50 pm. Im Falle von WGMs als optische Resonanzmoden, die in suspendierten Polystyrolpartikeln von 10 µm Durchmesser im Bereich von 500 nm angeregt werden, liegt die beobachtbare Verschiebung der Moden für eine Monolage eines Proteins typischerweise bei etwa 0.4 nm. Dies ergibt sich aus dem Zusammenhang Δλ/λ = ΔR/R (Gleichung 1) zwischen der Wellenlänge λ der optischen Resonanzmode, dem Radius des Mikropartikels R sowie deren Änderungen Δλ und ΔR für den Fall λ = 500 nm, R = 5000 nm sowie einer typischen effektiven Schichtdicke ΔR einer Proteinmonolage auf dem Mikropartikel von 4 nm (die effektive Schichtdicke hängt typischerweise sowohl vom Protein selbst als auch von den Untersuchungsbedingungen, wie dem Elektrolytgehalt, ab).

Demzufolge sollte die optische Auflösung bei mindestens einem Achtel dieses Werts liegen, um eine Beobachtung des Adsorptionsprozesses zu gewährleisten.

Der Detektor 7 wandelt das analysierte Licht des Spektral-Analysators 6 in ein elektronisch verarbeitbares Signal um. Je nach Ausführung des Spektral-Analysators 6 eignen sich hierzu insbesondere Bildsensoren (2-dimensionale Pixel-Arrays), Zeilen-Kameras (1-dimensionale Pixel-Arrays), Photodioden (null-dimensionale Pixel-Arrays) oder andere punktförmige Sensoren.

Die digitale Signalverarbeitung 10 verarbeitet das vom Detektor 6 gewandelte Signal. Falls nicht schon intern in Spektral-Analysator 6 oder Detektor 7 geschehen, wird hier zunächst der Zusammenhang zwischen Signal-Intensität und Wellenlängenposition hergestellt. Aus diesem Primär-Signal können dann durch geeignete Algorithmen die Positionen, Bandbreiten und Aufspaltungen der im analysierten Spektralbereich auftretenden optischen Resonanzmoden detektiert und quantifiziert werden. Durch die Wahl und Auslegung der benutzten Algorithmen kann die Vorrichtung 100 die Analyse sogar in Echtzeit vornehmen und so dem Nutzer des Systems 100 bereits während der Messung zur Verfügung gestellt werden kann. Dieser kann damit erstmals auf die noch laufende Messung reagieren bzw. Einfluss nehmen.

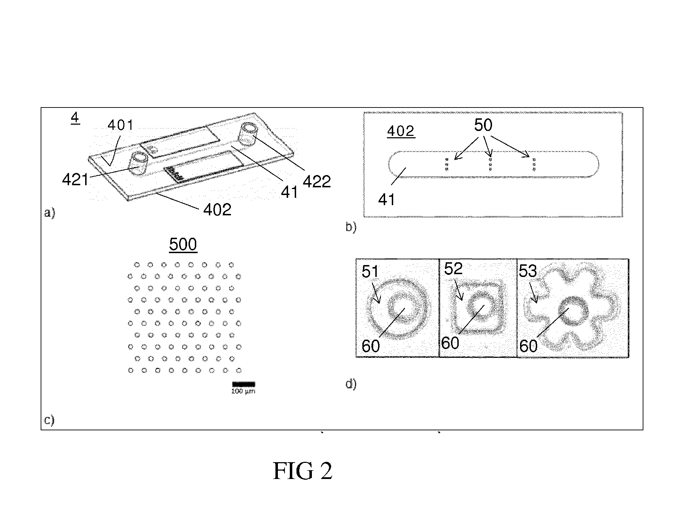

Figur 2 zeigt in der Teilfigur a eine perspektivische Darstellungen eines Mikrochip 4 wie er in der oben mit Bezug zur Figur 1 erläuterten Vorrichtung 100 verwendet werden kann. Der Mikrochip 4 weist einen Mikrokanal 41 auf. Der Boden des Mikrokanals 41 wird in dem exemplarischen Ausführungsbeispiel von einem transparenten Substrat (Objektträger) 402 gebildet, auf das eine Schicht mit verschiedenen Lochstrukturen 50 bis 52 aufgebracht wurde, die in den Teilfiguren b bis d dargestellt werden.

Diese Strukturen können beispielsweise photolithographisch erzeugt werden: Hierfür wird der Objektträger 402 mit einem Photolack (SU-8) beschichtet (mittels Spincoating oder Trockenverfahren), der durch eine Maske teilweise belichtet wird, sodass nach Herauslösen der unbelichteten Bereiche Löcher in der (in den belichteten Bereichen ausgehärteten) Photolackschicht zurückbleiben. Form und Größe der Löcher werden somit durch die Struktur der Maske festgelegt. Teilfigur d) zeigt Bilder von drei verschiedenen Lochformen: kreisrunde 51, quadratische 52 und sternförmige Löcher 53 mit jeweils einem im Mikrokanal 41 gehaltenen Partikel 60.

Teilfigur c) zeigt eine hexagonale Anordnung 500 einer Vielzahl von Löchern am Boden des Mikrokanals.

Während sich bei kreisrunden Löchern eine relativ große Kontaktfläche zwischen Lochwand und kugelförmigem Partikel ergeben kann, wird die mögliche Kontaktfläche über die Quadrate hin zu den Sternen minimiert. Dadurch kann sichergestellt werden, dass die Lochwand keinen maßgeblichen Einfluss auf die Ausbildung der optischen Resonanzmoden in den Mikropartikeln hat und darüber hinaus eine größere Partikeloberfläche zur Adsorption des gesuchten Liganden zu Verfügung steht.

Die Größe der Haltestrukturen eines Mikrochips kann variieren, so dass im Mikrochip Haltestrukturen für verschiedene Partikelgrößen vorhanden sein können, in denen jeweils genau ein Partikel Platz findet und damit gehalten werden kann.

Der Kanal 4 entsteht typischerweise durch Aufkleben des Lochstrukturbodens auf einen selbstklebenden Kunststoffchip 401. In diesem Chip 401 ist eine entsprechende Einkerbung (mit exemplarischer Höhe 20-400 µm, Breite 5 mm und Länge 50 mm) eingebracht, die die Seitenwände und die Decke des Mikrokanals 4 darstellt. In der Decke des Chips 401 sind außerdem zwei Luer-Anschlüsse 421, 422 eingelassen, die es ermöglichen, das entstehende Volumen (5µL-200µL) zu durchspülen, sodass es als Mikrokanal, in dessen Boden sich Löcher befinden, verwendet werden kann. Je nach Anzahl und Anordnung der Haltestrukturen kann die Breite des Mikrokanals auch deutlich geringer sein, z.B. geringer als etwa 1 mm oder sogar nur etwa 100 µm.

Das Fangen der Partikel erfolgt nach Injizieren einer Partikelsuspension in den Mikrokanal 4 während einer anschließenden Sedimentationsphase. Ein Partikel, das sich über einem Loch befindet, senkt sich nach ausreichender Wartezeit (je nach Dichte der Partikel einige Minuten) in dieses Loch hinein. In der folgenden Spülphase werden die Partikel in den Löchern festgehalten, alle anderen werden aus dem Kanal entfernt. Der Vorgang kann gegebenenfalls für Multiplexing-Verfahren mit weiteren, anders funktionalisierten Partikelchargen wiederholt werden.

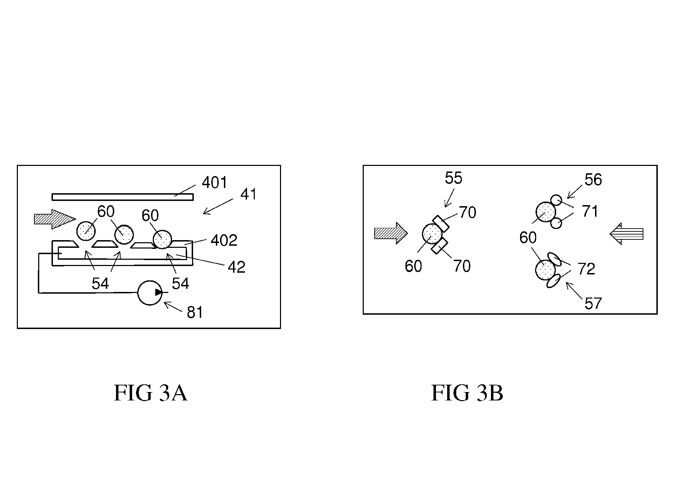

Figur 3A demonstriert in einer Querschnittsansicht durch einen Teil eines Mikrokanals 41, der zwischen einem die Kanaldecke bildenden Objektträger 401 und einem den Kanalboden bildenden Kunststoffchip 402 eines Mikrochips gebildet ist, den Prozess des hydrodynamischen Führens eines Partikels 60 in eines von drei exemplarischen trichterförmigen Durchgangslöchern 54, die als Haltestrukturen dienen können. In der Aufsicht sind die Durchgangslöcher typischerweise kreisförmig. Durch Verwendung trichterförmige Haltestrukturen 54 kann die Untersuchungsposition (Halteposition) des Partikels im Mikrochip sehr genau vorbestimmt werden. In dem exemplarischen Ausführungsbeispiel gemäß Figur 3A kann die Partikelsuspension mit einer ersten nichtgezeigten Pumpe in den Mikrokanal 41 eingespült werden. Die Hauptströmung im Mikrokanal 41 wird durch den gestreiften Pfeil symbolisiert. Wenn das Partikel 60 mit der Hauptströmung in die Nähe der Durchgangslöcher 54 gelangt ist, kann eine zweite Pumpe 81 aktiviert werden, die Flüssigkeit aus einem unterhalb des Mikrokanals 41 angeordneten und mit diesem über die Durchgangslöcher 54 fluidisch gekoppelten weiteren Mikrokanal 42 saugt. Dadurch wird das Partikel 60 in eine der Durchgangslöcher 54 geführt. Im Vergleich zu einem Führen des Mikropartikels 60 über die Hauptströmung und Sedimentieren in das Durchgangsloch 54, wird durch das Ansaugen mittels der Pumpe 81 ein schnelleres und sichereres Beladen der Haltestrukturen 54 ermöglicht. Außerdem können die beladenen Haltestrukturen nach erfolgte Messung über eine Umkehrung der Strömungsrichtung der Pumpe 81 einfacher und sicherer wieder entladen werden.

Figur 3B zeigt drei exemplarische Haltestrukturen 55-57, die jeweils von zwei am Boden des Mikrokanals angeordneten und voneinander beabstandeten Barrieren 70, 71, 72 gebildet werden und mit einem jeweiligen gehaltenen Partikel 60 in einer Aufsicht bzw. einem Querschnitt parallel zum Boden des Mikrokanals dargestellt sind. Die Barrieren reichen typischerweise bis nahe an die Decke des Mikrokanals (z.B. bis einige µm an die Decke), der in diesem Fall eine relativ geringe Höhe von bspw. 20 µm bis 100 µm haben kann. Wird durch den Mikrokanal eine Partikelsuspension in Richtung des schräggestreiften Pfeils geströmt, kann sich je Haltestruktur 55 bis 57 ein Partikel 60 verfangen, welches dann in der jeweiligen vom Mikrochip vordefinierten Position gemessen und weiter von Analyt- bzw. Spüllösungen umströmt werden kann. Nach Beendigung der Messung kann während typischerweise 1-3 Zyklen die Strömungsrichtung kurzzeitig, z.B. für einige Sekunden, umgekehrt (horizontal gestreifter Pfeil) und die Partikel so aus dem Mikrokanal und dem Mikrochip entfernt werden. Wie aus Fig. 3B ersichtlich ist, können die Barrieren 70, 71, 72 im Querschnitt parallel zum Boden des Mikrokanals kreisförmig, rechteckig oder elliptisch sein, wobei die maximale Ausdehnung der Barrieren 70, 71, 72 etwa dem Durchmesser der Partikel 60 entsprechen kann, kleiner als der Partikeldurchmesser sein kann, aber auch bis etwa dem zweifachen Partikeldurchmesser entsprechen kann. Die Verwendung von Barrieren 70, 71, 72, deren maximale Ausdehnung im Querschnitt in einem Bereich bis etwa zum doppelten Durchmesser der Partikel 60 liegt, ermöglicht ein einfaches und zuverlässiges Partikelentladen. Daher kann der Mikrochip mehrfach verwendet werden.

Im Folgenden werden zwei typische Messabläufe geschildert, wobei zunächst die automatische Vermessung eines oder mehrerer Mikropartikel-Sensoren und danach das sogenannte Multiplexing, d.h. die Simultan-Detektion mehrerer spezifischer Bindungsereignisse unterschiedlicher Liganden, beschrieben wird.

Gemäß einem Ausführungsbeispiel wird zur automatischen Vermessung eines oder mehrerer Mikropartikel-Sensoren mit der oben mit Bezug zur Figur 1 erläuterten Vorrichtung zur Bestimmung einer Kinetik bzw. eines Sensogramm zunächst je ein Mikropartikel in eine jeweilige Haltestruktur des Mikrochips geführt und dort gehalten.

Anschließend können die Haltestrukturen auf gehaltene Partikel gescannt werden, was über eine Messung der Partikelemission erfolgen kann.

Für gefundene Partikel kann nun die Untersuchungsposition (Messposition) genauer bestimmt (in x, y & z) werden, was durch Maximierung der Signalintensität der Emission erfolgen kann.

Die genauer bestimmte jeweilige Untersuchungsposition wird gespeichert, so dass sie später wieder angefahren werden kann, während oder nachdem der Mikrokanal mit Analytlösung gespült wurde.

Nach Aufnehmen des jeweiligen Emissionsspektrums (Auslesen des CCD-Sensors bzw. Gitterscan bei Photodiode) kann für jedes gehaltene Partikel ein Sensogramm wie folgt ermittelt werden: In dem optional geglätteten und/oder beschnittenen Datensatz des Emissionsspektrums (Intensität als Funktion der Wellenlänge) werden über automatische Peak-Finder-Routinen, die z.B. Lorentzprofile als Fitfunktionen für die Moden benutzen, die Resonanzmoden bestimmt (Peakwellenlänge, Modenbreiten und -amplitude) und als Funktion der Zeit ausgewertet und/oder dargestellt (Sensogramm). Optional kann dabei eine arithmetische Mittelung der Sensogramme der einzelnen Partikel erfolgen. Aus dem zeitlichen Verlauf der partikelbezogenen Sensogramme bzw. des gemittelten Sensogramms kann dann eine Kinetik der Wechselwirkung zwischen Analyt und Partikel und/oder die Absolutmenge des Adsorbats (bspw. nach Gleichung 1) bestimmt werden.

Gemäß einem Ausführungsbeispiel werden für das Multiplexing (Simultandetektion spezifischer Bindungsevents) nacheinander mehrere Suspensionen von Partikelsorten mit unterschiedlichen spezifisch-aktiven Oberflächen in den Mikrokanal injiziert, und einige der Partikel in entsprechende Haltestrukturen geführt und gehalten.

Danach bzw. zwischen den Injektionen der Suspensionen können, wie oben beschrieben, die Haltestrukturen auf gehaltene Partikel gescannt und für diese die jeweilige Untersuchungsposition genauer bestimmt und gespeichert werden.

Nun kann eine Pufferlösung in den Mikrokanal injiziert werden und ein jeweiliges Referenzspektrum (erstes Emissionsspektrum) der gehaltenen Partikel gemessen werden.

Anschließend kann eine Referenz-Peakpositionen einer jeweiligen ersten Resonanzmode für jedes einzelne Partikel bestimmt werden.

Nach einer Injektion einer Probe (Analytlösung) und einer optionalen Inkubationsphase kann eine erneute Vermessung der gehaltenen Partikel erfolgen (Bestimmen eines jeweiligen zweiten Emissionsspektrums der gehaltenen Partikel).

Nun können Peakpositionen von korrespondierenden Resonanzmoden in den zweiten Emissionsspektren und daraus eine Modenverschiebung gegenüber der jeweiligen Referenz-Peakpositionen bestimmt werden.

Nach einer optionalen arithmetischen Mittelung der Modenverschiebung für jede der verschiedenen Partikelsorten kann über einen Vergleich mit entsprechenden Grenzwerten bestimmt werden, ob ein jeweiliger Analyt in der Probe vorhanden war oder nicht und das entsprechende Ergebnis ausgegeben werden, z.B. als Testbericht auf den jeweiligen Antikörper (positiv/negativ).

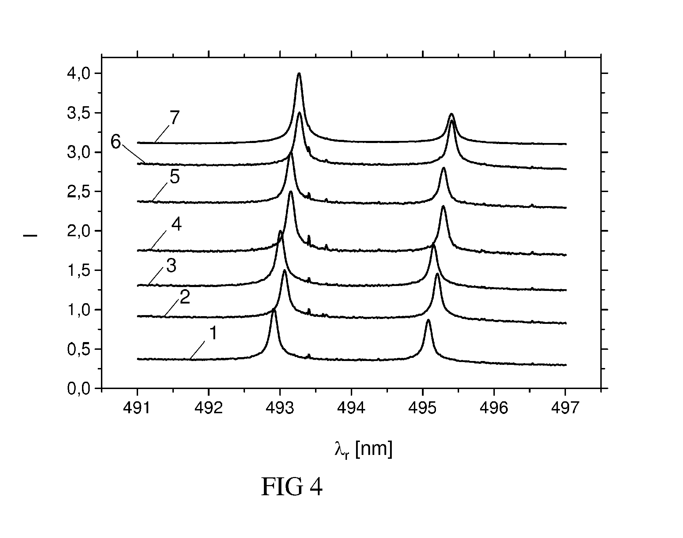

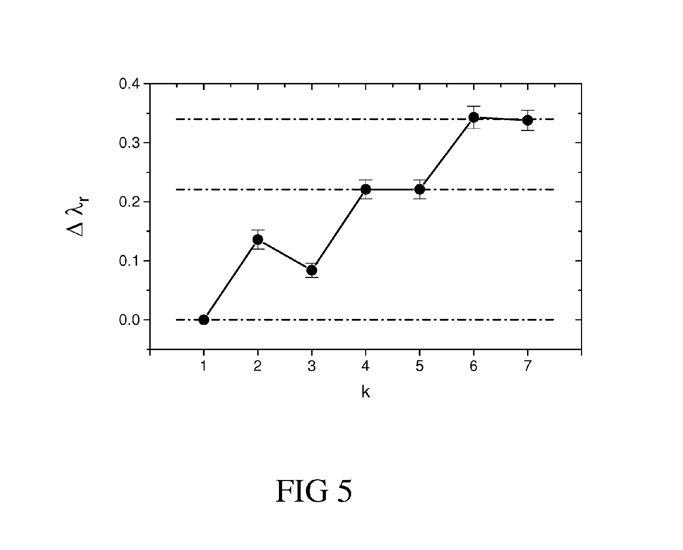

Mit Bezug zu den Figuren 4 und 5 werden nun Messungen zur Bestimmung von spezifischen Streptavidin-Biotin-Wechselwirkungen an Polystyrolpartikeln (PS-Partikeln) beschrieben, die einzeln in im Boden eines Mikrokanals gebildeten lochförmigen Haltestrukturen eines Mikrokanals eines Mikrochips gehaltenen wurden.

Die PS-Partikel mit einem Durchmesser von 10 +/- 0,3 µm wurden mit Coumarin 6 angefärbt und auf der Oberfläche kovalent Biotin gekoppelt (biotinyliert). Der an das oben mit Bezug zur Fig. 1 beschriebene Messsystem angeschlossene Mikrochip wurde über Nacht mit BSA passiviert und ausgiebig mit Wasser und anschließend mit PBS-Puffer gespült.

Danach wurden 10 µl einer 1%-igen Suspension der biotinylierten PS-Partikel in den Mikrochip gespült und die Strömung gestoppt. Nach einer Sedimentation der Partikel in die Löcher des Mikrochips wurden die überstehenden PS-Partikel mit PBS-Puffer ausgespült. Von den sedimentierten PS-Partikeln wurde das in Fig. 4 gezeigte erste Fluoreszenzemissionsspektrum 1 (Kurvenindex k=1) aufgenommen (WGM-Spektrum), um die Verwendbarkeit der modifizierten Partikel zu verifizieren. In Fig. 4 sind die Intensitäten I der WGM-Spektren in relativen Einheiten als Funktion der Wellenlänge λᵣ in einem Wellenlängenausschnitt mit zwei Resonanzmoden dargestellt.

Im Anschluss wurden 2 mL einer Streptavidin-Lösung durch den Mikrochip gespült, 10 min inkubiert und durch frische Streptavidin-Lösung ersetzt. Diese wurde nach weiteren 10 min mit 2 ml PBS-Puffer weggespült und dann ein zweites Fluoreszenzemissionsspektrum 2 aufgenommen (Kurvenindex k=2). Die deutliche Signalverschiebung (Figur 5) weist auf eine starke Adsorption des Streptavidins hin. Figur 5 zeigt die gemittelte Lageverschiebung der in Fig. 4 dargestellten zwei Resonanzmoden des zweiten und weiteren Fluoreszenzemissionsspektrums (k>1) relativ zum ersten Fluoreszenzemissionsspektrum (k=1) für ein gehaltenes PS-Partikel.

Zur Entfernung unspezifisch gebundenen Streptavidins wurde das gehaltene PS-Partikel 5 min mit Acetat-Puffer bei pH 4 behandelt und dann mit PBS-Puffer gespült. Das weitere Spektrum (k=3) zeigt anhand der Verschiebung in Fig. 5, dass ein Teil des Streptavidins wieder abgewaschen wurde. Sodann wurde die Oberfläche des PS-Partikels zur Verhinderung von unspezifischen Bindungen mit BSA Lösung geblockt und erneut mit PBS Puffer gespült. Die beiden ermittelten Resonanzpeaks haben sich langwellig verschoben (k=4), was eine Bedeckung der Oberfläche mit BSA anzeigt. Diese Prozedur wurde erneut ausgeführt, wobei sich das Spektrum nun nicht mehr verändert (k=5), d.h. es wird kein weiteres BSA unspezifisch adsorbiert.

Es folgte eine Inkubation des Partikels mit BSA, welches zuvor mit Biotin gelabelt wurde. Der Labelgrad beträgt durchschnittlich 1,5 Biotineinheiten pro BSA Molekül. Nach dem Wegwaschen der BSA-Biotinlösung mit PBS Puffer zeigt die Verschiebung der Peaks (k=6; Figur 5) eine spezifische Adsorption des BSA-Biotins an den Streptavidineinheiten. Abschließend wurde der Aufbau erneut mit Acetatpuffer pH 4 und mit PBS Puffer gespült. Die unveränderte Lage des Resonanzspektrums (k=7) zeigt die Stabilität des Messsystems gegenüber weiteren Spülschritten. Das Messsystem ist damit in der Lage, robust, zuverlässig und hochempfindlich Analyten über die Messung im Mikrochip gehaltener Partikel und einen anschließenden Vergleich von Emissionsspektren zu detektieren.

Die verwendeten Verfahren zur labelfreien Detektion eines Analyten umfassen in einem Ausführungsbeispiel die folgenden Schritte: Einströmen eines suspendierten Partikels in einen Mikrokanal eines Mikrochips, Führen des suspendierten Partikels in eine Haltevorrichtung des Mikrochips und Halten des suspendierten Partikels in der Haltevorrichtung, optisches Anregen des in der Haltevorrichtung gehaltenen Partikels und Messen eines ersten Emissionsspektrums des gehaltenen Partikels, Bestimmen einer ersten Resonanzmode des Partikels im ersten Emissionsspektrum, Zuführen eines Analyten zu dem in der Haltevorrichtung gehaltenen Partikel mittels einer Strömung, erneutes optisches Anregen des in der Haltevorrichtung gehaltenen Partikels und Messen eines zweiten Emissionsspektrums des gehaltenen Partikels, Bestimmen einer zur ersten Resonanzmode des Partikels korrespondierenden zweiten Resonanzmode des Partikels im zweiten Emissionsspektrum, Vergleichen der zweiten Resonanzmode mit der ersten Resonanzmode, um den Analyten zu detektieren. Anschließend kann das zur Messung benutzte Partikel aus der Haltevorrichtung wieder entfernt werden. Typischerweise umfasst das Vergleichen ein Bestimmen einer Lageverschiebung, einer Änderung einer Halbwertsbreite und/oder einer Aufspaltung der zweiten Resonanzmode relativ zur ersten Resonanzmode.

Bei der Haltevorrichtung kann es sich um eine im oder am Mikrokanal angeordnete mechanische Haltestruktur handeln, bspw. eine lochförmige und/oder trichterförmige Haltestruktur, und/oder um eine im oder am Mikrokanal angeordnete Haltevorrichtung, über die eine feldvermittelte Kraftwirkung auf das Partikel ausgeübt werden kann, bspw. eine Anordnung von Mikroelektroden.

Die nachfolgenden Ansprüche stellen einen ersten, nicht bindenden Versuch dar, die Erfindung allgemein zu definieren.

## Patentansprüche

1. Verfahren zur labelfreien Detektion eines Analyten, umfassend:
- Einströmen eines suspendierten Partikels (60) in einen Mikrokanal (41) eines Mikrochips (4);
- Führen des suspendierten Partikels in eine Haltestruktur (50 -56) im Mikrochip und Halten des suspendierten Partikels in der Haltestruktur;
- Optisches Anregen des in der Haltestruktur gehaltenen Partikels und Messen eines ersten Emissionsspektrums des gehaltenen Partikels;
- Bestimmen einer ersten Resonanzmode des Partikels im ersten Emissionsspektrum;
- Zuführen eines Analyten zu dem in der Haltestruktur gehaltenen Partikel mittels einer Strömung;
- Erneutes optisches Anregen des in der Haltestruktur gehaltenen Partikels und Messen eines zweiten Emissionsspektrums des gehaltenen Partikels;
- Bestimmen einer zur ersten Resonanzmode des Partikels korrespondierenden zweiten Resonanzmode des Partikels im zweiten Emissionsspektrum; und
- Bestimmen einer Lageverschiebung, einer Änderung einer Halbwertsbreite und/oder einer Aufspaltung der zweiten Resonanzmode relativ zur ersten Resonanzmode.

2. Verfahren nach Anspruch 1, wobei das suspendierte Partikel in einer vom Mikrochip vorbestimmten Untersuchungsposition gehalten wird.

3. Verfahren nach Anspruch 1 oder 2, wobei das Führen des suspendierten Partikels mindestens einen der Folgenden Schritte umfasst:
- Anströmen des suspendierten Partikels gegen eine im Mikrokanal angeordnete trichterförmige Haltestruktur;
- Sedimentieren des suspendierten Partikels in eine die Haltestruktur bildende Vertiefung oder ein Loch des Bodens des Mikrokanals;
- Hydrodynamisches Führen des suspendierten Partikels in ein die Haltestruktur bildendes Loch eines Bodens des Mikrokanals;
- Erzeugen eines inhomogenen elektrischen Feldes im Mikrokanal;
- Erzeugen eines Ultraschallfeldes im Mikrokanal;
- Erzeugen eines fokussierten elektromagnetischen Feldes im Mikrokanal;
und
- Erzeugen eines Magnetfeldes im Mikrokanal.

4. Verfahren nach einem der Ansprüche 1 bis 3, wobei das erste und/oder das zweite Emissionsspektrum mit einem spektralen Auflösungsvermögen von zumindest 10⁴ gemessen werden.

5. Verfahren nach einem der Ansprüche 1 bis 4, weiter umfassend:
- Erneutes optisches Anregen des in der Haltestruktur gehaltenen Partikels und Messen eines weiteren Emissionsspektrums des gehaltenen Partikels;
- Bestimmen einer zur ersten Resonanzmode und/oder zweiten Resonanzmode des Partikels korrespondieren weiteren Resonanzmode des Partikels im weiteren Emissionsspektrum;
- Bestimmen einer Lageverschiebung, einer Änderung einer Halbwertsbreite und/oder einer Aufspaltung der weiteren Resonanzmode relativ zur ersten Resonanzmode und/oder zweiten Resonanzmode; und/oder
- Entfernen des in der Haltestruktur (50-56) gehaltenen Partikels (60) aus der Haltestruktur (50-56) nach dem Messen des zweiten Emissionsspektrums oder nach dem Messen des weiteren Emissionsspektrums.

6. Verfahren nach Anspruch 5, weiter umfassend Bestimmung einer Kinetik der Adsorption des Analyten an dem in der Haltestruktur gehaltenen Partikel.

7. Verfahren nach einem der Ansprüche 1 bis 6, weiter umfassend Bestimmung einer Menge des auf dem in der Haltestruktur gehaltenen Partikel adsorbierten Analyten.

8. Verfahren nach einem der Ansprüche 1 bis 7, wobei mehrere suspendierte Partikel in jeweiligen Haltestrukturen im Mikrochip geführt und gehalten werden, und von denen ein jeweiliges erstes und zweites Emissionsspektrum gemessen werden.

9. Verfahren nach einem der Ansprüche 1 bis 8, weiter umfassend vor dem Messen des ersten Emissionsspektrums:
- Detektieren des in der Haltestruktur gehaltenen Partikels; und/oder
- Bestimmen einer Position des in der Haltestruktur gehaltenen Partikels.

10. Vorrichtung (100) zur labelfreien Detektion eines Analyten, umfassend:
- eine Fluidikeinheit umfassend eine Dosiereinheit (13, 14);
- einen Mikrochip (4) umfassend einen Mikrokanal (41), der mit der Dosiereinheit (13, 14) derart verbundenen ist, dass eine Lösung mit dem Analyten und eine Suspension von Partikeln (60) in den Mikrokanal (4) gefördert werden kann, und eine im oder am Mikrokanal (4) angeordnete Haltestruktur (50 -56) für die Partikel;
- eine Lichtquelle (1) zur Anregung einer Resonanzmode eines in der Haltestruktur (50 -56) gehaltenen Partikels (60);
- einen Spektralanalysatoreinheit (6, 7) zur Messung eines Emissionsspektrums des in der Haltestruktur (50 -56) gehaltenen Partikels (60); und
- eine Steuer- und Auswerteeinheit (9, 10), die mit der Lichtquelle (1), der Spektralanalysatoreinheit (6, 7) und der Fluidikeinheit verbunden ist und eingerichtet ist, eine Resonanzmode im Emissionsspektrum zu bestimmen.

11. Vorrichtung nach Anspruch 10, wobei die mindestens eine Haltestruktur ein Loch mit einem Durchmesser von weniger als 30 µm in einem Boden des Mikrokanals und/oder zwei am Boden des Mikrokanals angeordnete und voneinander beabstandete Barrieren (70, 71, 72), umfasst.

12. Vorrichtung nach Anspruch 10 oder 11, wobei der Mikrochip mehrere ein Raster bildende Haltestrukturen umfasst.

13. Vorrichtung nach einem der Ansprüche 10 bis 12, wobei die Steuer- und Auswerteeinheit (9, 10) eingerichtet ist, ein Verfahren mit den Schritten:
- Einströmen der Suspension in den Mikrokanal;
- Führen des suspendierten Partikels in die Haltestruktur und Halten des suspendierten Partikels in der Haltestruktur;
- Optisches Anregen des in der Haltestruktur gehaltenen Partikels und Messen eines ersten Emissionsspektrums des in der Haltestruktur gehaltenen Partikels;
- Bestimmen einer ersten Resonanzmode des Partikels im ersten Emissionsspektrum;
- Zuführen des Analyten zu dem in der Haltestruktur gehaltenen Partikel mittels einer Strömung der Lösung;
- Erneutes optisches Anregen des in der Haltestruktur gehaltenen Partikels und Messen eines zweiten Emissionsspektrums des in der Haltestruktur gehaltenen Partikels;
- Bestimmen einer zur ersten Resonanzmode des Partikels korrespondieren zweiten Resonanzmode des Partikels im zweiten Emissionsspektrum;
- Entfernen des in der Haltestruktur (50-56) gehaltenen Partikels (60) aus der Haltestruktur (50-56) nach dem Messen des zweiten Emissionsspektrums; und
- Bestimmen einer Lageverschiebung, einer Aufspaltung und/oder einer Änderung einer Halbwertsbreite der zweiten Resonanzmode relativ zur ersten Resonanzmode.

14. Vorrichtung nach einem der Ansprüche 10 bis 13, wobei die Spektralanalysatoreinheit (6, 7) einen Gittermonochromator mit einer Liniendichte von mindestens 1200/mm als Spektralanalysator (6) und einen Bildsensor (7) mit mindestens 1000 Pixeln und mit einer Pixelbreite von höchstens 24 µm als Detektor umfasst; wobei die Spektralanalysatoreinheit (6, 7) einen Gittermonochromator mit einer Liniendichte von mindestens 1200/mm als Spektralanalysator (6), eine Photodiode (7) als Detektor und einen zwischen dem Gittermonochromator und der Photodiode angeordneten Spalt von maximal 100 µm umfasst; oder wobei die Spektralanalysatoreinheit (6, 7) ein Etalon umfasst.

15. Vorrichtung nach einem der Ansprüche 10 bis 14, weiter umfassend:
- eine Optik (3, 5) zum Einkoppeln des Lichts der Lichtquelle (1) in den Mikrochip (4) und zum Weiterleiten von emittierten Licht des in der Haltestruktur gehaltenen Partikels zum Spektralanalysator (6, 7);
- eine mit der Steuer- und Auswerteeinheit (9, 10) verbundene motorisierte Verstelleinheit (8) zum Verfahren des Mikrochips relativ zur Optik (3, 5); und/oder
- einen Shutter (2), der das Licht der Lichtquelle (1) blockieren kann.
